# EUROPEAN PATENT APPLICATION

(11) **EP 4 434 514 A1**
(43) Date of publication of application: **25.09.2024**
(21) Application number: 22895927.6
(22) Date of filing: 04.11.2022
(51) Int. Cl.: A61K 9/00, A61K 9/50, A61K 9/28, A61K 9/20, A61K 9/16

(54) **OSMOTIC AGENT COMPOSITION COMPRISING ACID-SENSITIVE INGREDIENT**

(30) Priority: 16.11.2021 KR 20210157454
(71) Applicant: Oddson Bio Co., Ltd., Seoul 02796 (KR)
(72) Inventor: CHUN, Myungkwan, Gwangju 61198 (KR); SEONG, Jeonga, Seoul 02603 (KR)
(74) Representative: SSM Sandmair
(86) International application number: PCT/KR2022/017263
(87) International publication number: WO 2023/090720

(57) **Abstract**

The present invention relates to an osmotic agent composition comprising an acid-sensitive polymer in a coating layer. More particularly, the present invention relates to an osmotic agent composition and a method for preparing same, the osmotic agent composition comprising an acid-sensitive polymer that can be dissolved or separated in an acidic condition so as to form a porous osmosis membrane on a coating layer of the osmotic agent composition.

## Description

### Technical Field

The present invention relates to an osmotic agent composition including an acid-sensitive ingredient. In particular, the present invention relates to an osmotic agent composition and a method for preparing the same, the osmotic agent composition including an acid-sensitive polymer that can be dissolved or separated in an acid condition so as to form a porous osmosis membrane on a coating layer of the osmotic agent composition that contains an active ingredient.

### Background Art

Among drug delivery systems, a drug delivery system using osmotic pressure is one of the most advanced technologies, and, since osmotic pressure is used as a main driving force for drug dissolution, is capable of controlling drug dissolution without being affected by food intake, pH of gastrointestinal tract, and the like.

Pioneers in the invention of formulations using osmotic pressure are Rose and Nelson. Rose and Nelson are Australian physiologists who were interested in delivering drugs to intestines of sheep and cattle, and the formulation they designed is formed with three reservoirs. In other words, the formulation is formed with a drug reservoir, a reservoir formed with excess salt acting as an osmagent, and a reservoir formed with water.

US Patent No. 3,604,417 discloses a technology of replacing an elastic isolation membrane of the formulation invented by Rose and Nelson with a moving piston. This technology was the first patent using osmotic pressure as a driving force for drug release, however, this formulation has a problem in that osmosis occurs as soon as water permeates a semi-permeable membrane. In other words, it was not able to be actually clinically applied due to the disadvantage of having to store the formulation in an empty state without water and manually introducing water only immediately before use.

US Patent No. 3,760,804 discloses a technology of allowing a formulation to operate by absorbing water from a surrounding environment without a water reservoir, however, despite the advantage of storing a drug for weeks or months before use after encapsulation, the formulation has a complex structure and it is difficult to commercialize this technology due to high manufacturing costs.

US Patent No. 3,995,631 describes a technology of tableting a drug having proper osmotic pressure using a tablet press machine by using the drug itself as an osmagent, and, after coating the tablet with a semi-permeable membrane, perforating one micro-hole. However, drugs with low solubility may not readily act as an osmagent, and there is a disadvantage of not being applicable to drugs with low drug solubility.

US Patent No. 4,327,725 discloses a formulation in which a core layer has a dual structure of a drug storage layer and a volume expansion layer, however, there is an inconvenience in that an expensive perforating device is required to drill a micro-hole for drug release.

Korean Patent No. 10-0192154 discloses a method of coating an active ingredient with a pH-sensitive polymer that protects the active ingredient at pH 6 and releases the active ingredient at pH 2. However, for this purpose, a monomer or a mixture of monomers producing the pH-sensitive polymer needs to be polymerized in an aqueous emulsion under the presence of a surfactant, and the aqueous emulsion of the pH-sensitive polymer obtained thereby needs to be attached to the active ingredient without intermediate isolation of the polymer. This leads to a disadvantage of relatively complex manufacturing process, and there is also no description of a case that applies this method to a formulation using osmotic pressure.

Korean Patent No. 10-2253393 describes polylactic acid-glycolic acid copolymer-based microparticles containing a pH-sensitive polymer, however, this uses a property of the DEAP unit in a pH-sensitive poly(Lys-DEAP) polymer having a nonionic (hydrophobic) property at normal pH (pH 7.4), but having an ionic (hydrophilic) property under an alveolar respiratory acidosis environment (acidic environment, pH 6.0 to 7.0) by the tertiary amine included in the DEAP being ionized. Due to the ionization of the DEAP, a water channel is created inside the microparticles, and water gets in between the PLGA particles, causing the collapse of the microparticles, and herein, a drug encapsulated inside the PLGA and the pH-sensitive poly(Lys-DEAP) polymer is slowly released through the water channel, which is different from a principle of the present invention in which a porous osmosis membrane is formed by dissolving an acid-sensitive polymer itself.

The inventors of the present invention have discovered that, when an acid-sensitive polymer dissolvable or separable under an acidic condition is introduced to a coating layer in a formulation using osmotic pressure, an osmotic agent composition having a new mechanism, which minimizes an initial delay in release while having a stable release effect, may be developed by the acid-sensitive polymer being dissolved in an acidic environment in vivo to form a porous osmosis membrane, and have completed the present invention.

The osmotic agent composition of the present invention is capable of preventing errors in the perforation location or perforation depth, which may occur during laser perforation, and clogging of a few micro-holes caused by food, and in addition thereto, is capable of reducing manufacturing costs and is readily mass produced, and therefore, is expected to be vastly utilized in the field of medicine in the future.

### DISCLOSURE

### Technical Problem

The present invention is directed to providing an osmotic agent composition including a semi-permeable membrane coating ingredient and an acid-sensitive polymer in a coating layer, thereby minimizing an initial delay in the release of a drug while having a stable release effect.

The present invention is also directed to providing an osmotic agent composition including an acid-sensitive polymer capable of reducing manufacturing costs and readily mass produced.

However, the problems to be solved by the present invention are not limited to the above-mentioned problems, and other problems not mentioned will be clearly understood by those skilled in the art from the following description.

### Technical Solution

In the following description, various specific details such as specific forms, compositions and processes are descried to fully understand the present invention. However, specific embodiments may be implemented with one or more of these specific details or other known methods and forms. In other instances, known processes and manufacturing technologies are not described as specific details in order not to unnecessarily obscure the present invention. Reference throughout the present specification to "one embodiment" or "an embodiment" means that a particular feature, form, composition or characteristic described in connection with the embodiment is included in on or more embodiments of the present invention. Accordingly, the status of "in one embodiment" or "embodiment" expressed in various places throughout the present specification does not necessarily refer to the same embodiment of the present invention. In addition, the particular feature, form, composition or characteristic may be combined in any suitable method in one or more embodiments.

Unless particularly defined in the specification, all scientific and technical terms used in the present specification have the same meaning as those commonly understood by those skilled in the art.

In order to achieve the object of the present invention, one embodiment of the present invention provides an osmotic agent composition including a core layer including an active ingredient, an osmagent and a polymer; and a coating layer including a semi-permeable membrane coating ingredient and an acid-sensitive polymer.

Hereinafter, each constitution will be described in detail.

The "acid-sensitive polymer" used in the present invention is defined as a polymer that is dissolvable under an acidic condition of lower than pH 7.

In one embodiment of the present invention, when the coating layer containing an acid-sensitive polymer is exposed to an acidic condition in vivo, the acid-sensitive polymer may be dissolved or ionized in the coating layer to form micropores in the coating layer.

According to one embodiment of the present invention, the acid-sensitive polymer that may be used in the present invention is, as a methacrylate copolymer, an amino methacrylate copolymer that is dissolvable under an acidic condition (Eudragit^{ⓡ} E) or a modified copolymer thereof. Specifically, the acid-sensitive polymer that may be used in the present invention may be a copolymer of butyl methacrylate, dimethylaminoethyl methacrylate and methyl methacrylate ((Eudragit)^{ⓡ}E PO), but is not limited thereto.

In one embodiment of the present invention, the acid-sensitive polymer may be included in an amount of 10% by weight to 90% by weight with respect to the total weight of the coating layer. When the acid-sensitive inorganic ingredient is included in an amount of greater than 90% by weight, the coating layer may not remain after a dissolution test, and when included in an amount of less than 10% by weight, desired effects of the present invention related to initial dissolution rate control may not be achieved.

According to one embodiment of the present invention, the semi-permeable membrane coating ingredient that may be used in the present invention may be one or more types of ingredients selected from the group consisting of cellulose acylate, cellulose diacylate, cellulose triacylate, cellulose acetate, cellulose diacetate, acetaldehyde dimethyl cellulose acetate, cellulose acetate ethyl carbamate and cellulose dimethylamino acetate, but is not limited thereto.

In one embodiment of the present invention, the semi-permeable membrane coating ingredient may be included in an amount of 10% by weight to 90% by weight with respect to the total weight of the coating layer.

In one embodiment of the present invention, an organic acid may be included in an amount of 10% by weight to 50% by weight with respect to the total weight of the coating layer. Herein, the organic acid is added into the coating layer and dissolved in a biofluid to locally create an acidic environment when the osmotic agent composition is administered to a body, and helps the acid-sensitive polymer to be dissolved or separated more readily in the coating layer.

In one embodiment of the present invention, the organic acid and the acid-sensitive polymer preferably have a ratio of 3:1 to 1:10. When the ratio is outside the above-mentioned range, the ratio of the acid-sensitive polymer capable of forming micropores decreases in the whole coating layer, which may suppress micropore formation.

According to one embodiment of the present invention, the organic acid that may be used in the present invention may be one or more types of pharmaceutically acceptable organic acids selected from the group consisting of citric acid, fumaric acid, tartaric acid, maleic acid, malic acid, succinic acid, oxalic acid, malonic acid, benzoic acid, mandelic acid, glutamic acid, ascorbic acid and hydrates thereof, but is not limited thereto.

In one embodiment of the present invention, the active ingredient is an ingredient selected from the group consisting of tofacitinib, apremilast, nintedanib and pirfenidone, or a pharmaceutically acceptable salt thereof.

Tofacitinib is generally known to be a useful therapy as an immunosuppressant for organ transplantation, xenotransplantation, lupus, multiple sclerosis, rheumatoid arthritis, psoriasis, psoriatic arthritis, type I diabetes and diabetic complications, cancer, asthma, atopic dermatitis, autoimmune thyroid disease, ulcerative colitis, Crohn's disease, Alzheimer's disease, leukemia and other indications for which immunosuppression is desired, and is also sold under the brand name of Xeljanz.

Apremilast is a generic name of Otezla approved by the FDA of the US in 2014 and marketed as a treatment for psoriatic arthritis and psoriasis, and is known as a psoriasis treatment of PDE4 (phosphodiesterase 4) inhibitor class that blocks activation of PDE4 enzyme responsible for inflammatory reactions instead of an interleukin inhibitor.

Nintedanib is a tyrosine kinase inhibitor and sold under the brand name of Ofev, and is reported to be applicable to various lung diseases including idiopathic pulmonary fibrosis and interstitial lung diseases.

Pirfenidone is a drug developed as a treatment for idiopathic pulmonary fibrosis, but has received attention by the recent development of its potential use in treating patients with chronic fibrosing interstitial lung diseases and heart failure with preserved ejection fraction (HFpEF).

Unless instructed otherwise herein, the terms "tofacitinib", "apremilast", "nintedanib" and "pirfenidone" used in the present invention need to be understood to include any pharmaceutically acceptable forms and salts of the compounds. The above-described compounds may be present in a crystalline or amorphous form.

In one embodiment of the present invention, the osmagent may be formed with one or more types of ingredients selected from the group consisting of water-soluble salts, sugars, organic acids and gelling agents.

According to one embodiment of the present invention, the water-soluble salt that may be used in the present invention may be one or more types of water-soluble salt ingredients selected from the group consisting of magnesium sulfate, magnesium chloride, calcium chloride, sodium chloride, lithium chloride, potassium sulfate, sodium carbonate, sodium sulfite, lithium sulfate, potassium chloride and sodium sulfate, but is not limited thereto.

In one embodiment of the present invention, the osmagent may be included in an amount of 1% by weight to 70% by weight with respect to the total weight of the core layer.

According to one embodiment of the present invention, the polymer that may be used in the present invention includes water-soluble or oil-soluble polymers, and may be one or more types of ingredients selected from the group consisting of sodium carboxymethyl cellulose, carboxymethyl cellulose, hydroxypropyl cellulose, hypromellose, methyl cellulose, hydroxyethyl cellulose, polyvinyl pyrrolidone, a vinyl pyrrolidone and vinyl acetate copolymer, polyethylene oxide, ethyl cellulose and wax, but is not limited thereto.

In one embodiment of the present invention, the polymer may be included in an amount of 10% by weight to 70% by weight with respect to the total weight of the core layer.

In one embodiment of the present invention, the osmotic agent composition of the present invention may have 2% or more of the active ingredient dissolved within 30 minutes in 900 mL of a pH 1.2 dissolution medium using a Korean Pharmacopoeia Dissolution Test Method (Method 2).

In one embodiment of the present invention, the osmotic agent composition of the present invention does not experience a phenomenon of delay in the dissolution after 30 minutes in 900 mL of a pH 1.2 dissolution medium using a Korean Pharmacopoeia Dissolution Test Method (Method 2). In other words, the osmotic agent composition of the present invention may minimize a phenomenon of initial delay in dissolution that existing osmotic agent compositions experience.

The composition according to the present invention may further include a moisture absorbing component. The moisture absorbing component may be one or more types selected from the group consisting of colloidal silicon dioxide, polyvinyl pyrrolidone, sodium lauryl sulfate, kaolin, bentonite, niacinamide, alumina and titanium dioxide, but is not limited thereto.

In one embodiment of the present invention, the coating layer may be included in an amount of 2% by weight to 30% by weight with respect to the total weight of the whole composition.

The composition of the present invention may further include a dissolution enhancing agent component. The dissolution enhancing agent component may be one or more types selected from the group consisting of polyvinyl pyrrolidone, polyethylene glycol, beta-cyclodextrin, polysorbate, sodium lauryl sulfate, alkyl ester, sorbitan monolaurate, sorbitan monostearate and sorbitan monopalmitate, but is not limited thereto.

The composition of the present invention may further include a plasticizer component. The plasticizer component may be one or more types selected from the group consisting of polyethylene glycol, triacetin, triethyl citrate, dibutyl sebacate, ethylene glycol monoacetate, ethylene glycol diacetate, triethyl phosphate and diethyl tartrate, but is not limited thereto.

The composition of the present invention may further include a diluting agent, a binding agent, a disintegrating agent, a foaming agent, an emulsifying agent, a pH regulating agent, a lubricant or a glidant component.

In the composition of the present invention, the coating layer may have a thickness of 1 micrometer to 1,000 micrometers. When the coating layer has a thickness of less than 1 micrometer, the coating layer may collapse due to the internal pressure generated in the tablet core layer, and when the thickness is greater than 1,000 micrometers, desired effects of the present invention related to initial dissolution rate control may not be achieved.

According to one embodiment of the present invention, the composition of the present invention may be dissolved in a hydrophilic solvent. The hydrophilic solvent that may be used in the present invention may be, for example, one or more types selected from the group consisting of water, ionized water, physiological saline, distilled water, purified water, sterilized purified water and C₁₋₄ alcohols, but is not limited thereto, and is preferably water.

The osmotic agent composition of the present invention has a stable release effect by preventing errors in the perforation location or perforation depth, which may occur during laser perforation, and clogging of a few micro-holes caused by food, is capable of increasing patient's medication convenience and compliance by minimizing an initial delay in release, is capable of reducing manufacturing costs, and is readily mass produced.

### Advantageous Effects

The present invention provides an osmotic agent composition that minimizes an initial delay in the release of a drug while having a stable release effect by including a semi-permeable membrane coating ingredient and an acid-sensitive polymer in a coating layer.

In addition, the present invention provides an osmotic agent composition including an acid-sensitive polymer capable of reducing manufacturing costs and readily mass produced.

### Brief Description of Drawings

FIG. 1 is a graph comparing dissolution rates of tofacitinib citrate, an active ingredient in Example 4 to Example 6 and Comparative Example 1, after performing a dissolution test for 2 hours in 900 mL of a pH 1.2 dissolution medium using a Korean Pharmacopoeia Dissolution Test Method (Method 2).
FIG. 2 is a photograph showing, after performing a dissolution test for 12 hours in 900 mL of a pH 1.2 dissolution medium using a Korean Pharmacopoeia Dissolution Test Method (Method 2), surface shapes of a tablet of Example 4 before/after performing the dissolution test.
FIG. 3 is a photograph showing an inner shape of the tablet of Example 4 after performing the dissolution test for 12 hours in 900 mL of a pH 1.2 dissolution medium using a Korean Pharmacopoeia Dissolution Test Method (Method 2).
FIG. 4 is a photograph showing, after performing a dissolution test for 12 hours in 900 mL of a pH 1.2 dissolution medium using a Korean Pharmacopoeia Dissolution Test Method (Method 2), surface shapes of a tablet of Comparative Example 2 before/after performing the dissolution test.
FIG. 5 is a photograph showing an inner shape of the tablet of Comparative Example 2 after performing the dissolution test for 12 hours in 900 mL of a pH 1.2 dissolution medium using a Korean Pharmacopoeia Dissolution Test Method (Method 2).
FIG. 6 is an image of the surface state of the tablet of Example 4 before performing the dissolution test observed using a scanning electron microscope (SEM).
FIG. 7 is an image of the surface state of the tablet of Example 4 after performing the dissolution test for 12 hours in 900 mL of a pH 1.2 dissolution medium using a Korean Pharmacopoeia Dissolution Test Method (Method 2) observed using a scanning electron microscope (SEM).
FIG. 8 is a graph comparing dissolution patterns of tofacitinib citrate, an active ingredient in Example 1, Example 3 and Comparative Example 2, after performing a dissolution test for 12 hours in 900 mL of a pH 1.2 dissolution medium using a Korean Pharmacopoeia Dissolution Test Method (Method 2).
FIG. 9 is a graph comparing dissolution patterns of tofacitinib citrate, an active ingredient in Example 2, in 900 mL of each dissolution medium of pH 1.2, pH 4.0 and pH 6.8 using a Korean Pharmacopoeia Dissolution Test Method (Method 2).
FIG. 10 is a graph comparing dissolution patterns of nintedanib esylate, an active ingredient in Example 7 to Example 9, in 900 mL of a pH 1.2 dissolution medium using a Korean Pharmacopoeia Dissolution Test Method (Method 2).
FIG. 11 is a graph comparing dissolution patterns of apremilast, an active ingredient in Example 10 to Example 12, in 900 mL of a pH 4.0 (including 1% sodium lauryl sulfate) dissolution medium using a Korean Pharmacopoeia Dissolution Test Method (Method 2).
FIG. 12 is a graph comparing dissolution patterns of pirfenidone, an active ingredient in Example 13 to Example 15, in 900 mL of a pH 1.2 dissolution medium using a Korean Pharmacopoeia Dissolution Test Method (Method 2).

### Best Mode

Hereinafter, examples of the present invention will be described in detail so that those skilled in the art may readily implement the present invention. The present invention may be embodied in various different forms, and is not limited to examples, experimental examples and the like described herein.

### Examples 1 to 15

A pharmaceutical composition including an acid-sensitive polymer and a semi-permeable membrane coating ingredient was prepared in accordance with ingredients and contents of Tables 1 and 2 using the following preparation method:
mixing any one ingredient of tofacitinib citrate, apremilast, nintedanib esylate and pirfenidone as an active ingredient, polyethylene oxide, polyethylene glycol 6000 and hypromellose 2910;
(2) adding magnesium stearate to the mixture of step (1) and mixing the result;
(3) tableting the mixture of step (2); and
(4) coating the tablet of step (3) with a coating layer including cellulose acetate, an amino methacrylate copolymer and polyethylene glycol 4000 or with a coating layer including cellulose acetate, an amino methacrylate copolymer, polyethylene glycol 4000 and citric acid hydrate.

**[Table 1]**

| Categ ory | Ingredient | Example (% by weight) | | | | | | | |
|---|---|---|---|---|---|---|---|---|---|
| | | 1 | 2 | 3 | 4 | 5 | 6 | 7 | 8 |
| Core Layer | Tofacitinib Citrate | 9.0 | 9.0 | 8.8 | 8.8 | 8.6 | 8.4 | | |
| | Nintedanib Esylate | | | | | | | 8.8 | 8.6 |
| | Polyethylene Oxide¹⁾ | 22.8 | 22.8 | 22.2 | 22.2 | 21.8 | 21.4 | 22.2 | 21.8 |
| | Polyethylene Glycol 6000 | 58.8 | 58.8 | 57.2 | 57.2 | 56.2 | 55.2 | 57.2 | 56.2 |
| | Hypromellose 2910 | 3.6 | 3.6 | 3.5 | 3.5 | 3.4 | 3.3 | 3.5 | 3.4 |
| | Magnesium Stearate | 1.0 | 1.0 | 1.0 | 1.0 | 1.0 | 1.0 | 1.0 | 1.0 |
| Coati ng Layer | Cellulose Acetate | 0.7 | 2.4 | 3.0 | 2.2 | 2.8 | 3.3 | 3.7 | 4.5 |
| | Amino Methacrylate Copolymer²⁾ | 3.6 | 0.7 | 3.0 | 3.7 | 4.5 | 5.3 | 2.2 | 2.7 |
| | Citric Acid Hydrate | - | 1.4 | 1.2 | 1.3 | 1.6 | 1.9 | 1.1 | 1.4 |
| | Polyethylene Glycol 4000 | 0.4 | 0.2 | 0.3 | 0.2 | 0.3 | 0.3 | 0.4 | 0.5 |
| Total Weight (mg) | | 197. 2 | 197. 2 | 202. 8 | 202. 8 | 206. 6 | 210. 3 | 202. 8 | 206. 6 |
| ¹⁾ Average molecular weight of polyethylene oxide is 200,000. | | | | | | | | | |
| ²⁾ Amino methacrylate copolymer is Eudragit EPO. | | | | | | | | | |

**[Table 2]**

| Categ ory | Ingredient | Example (% by weight) | | | | | | |
|---|---|---|---|---|---|---|---|---|
| | | 9 | 10 | 11 | 12 | 13 | 14 | 15 |
| Core Layer | Nintedanib Esylate | 8.4 | | | | | | |
| | Apremilast | | 24.2 | 23.7 | 23.3 | | | |
| | Pirfenidone | | | | | 8.8 | 8.6 | 8.4 |
| | Polyethylene Oxide¹⁾ | 21.4 | 18.1 | 17.8 | 17.5 | 22.2 | 21.8 | 21.4 |
| | Polyethylene Glycol 6000 | 55.2 | 46.7 | 45.8 | 45.0 | 57.2 | 56.2 | 55.2 |
| | Hypromellose 2910 | 3.3 | 2.8 | 2.8 | 2.7 | 3.5 | 3.4 | 3.3 |
| | Magnesium Stearate | 1.0 | 0.8 | 0.8 | 0.8 | 1.0 | 1.0 | 1.0 |
| Coati ng Layer | Cellulose Acetate | 5.4 | 3.7 | 4.5 | 5.4 | 3.7 | 4.5 | 5.4 |
| | Amino Methacrylate Copolymer²⁾ | 3.2 | 2.2 | 2.7 | 3.2 | 2.2 | 2.7 | 3.2 |
| | Citric Acid Hydrate | 1.6 | 1.1 | 1.4 | 1.6 | 1.1 | 1.4 | 1.6 |
| | Polyethylene Glycol 4000 | 0.5 | 0.4 | 0.5 | 0.5 | 0.4 | 0.5 | 0.5 |
| Total Weight (mg) | | 210.3 | 248.4 | 253.0 | 257.6 | 202.8 | 206.6 | 210.3 |
| ¹⁾ Average molecular weight of polyethylene oxide is 200,000. | | | | | | | | |
| ²⁾ Amino methacrylate copolymer is Eudragit EPO. | | | | | | | | |

### Comparative Example 1

Comparative Example 1 was prepared in accordance with the ingredients and the contents of Example 2 except for the coating layer using the following preparation method:
mixing tofacitinib citrate, polyethylene oxide, polyethylene glycol 6000 and hypromellose 2910;
(2) adding magnesium stearate to the mixture of step (1) and mixing the result; and
(3) tableting the mixture of step (2).

### Comparative Example 2

Xeljanz XR extended-release 11 milligram (seller: Pfizer Pharmaceuticals Korea Co., Ltd.), an osmotic formulation using a separate perforating process using a perforating device, was used as Comparative Example 2.

### Experimental Example 1. Hardness of Pharmaceutical Composition Including Acid-Sensitive Ingredient and Osmotic Ingredient

Hardness of each of the tablets prepared in Examples was measured using a hardness measuring device. The measurement was performed 4 times, and the measurement results are shown in Table 3. As shown in Table 3, average hardness of Examples measured had values between 9.5 kp and 14.8 kp. In addition, as a result of measuring friability of these Examples, it was seen that, in all measured Examples, the values were less than 1.0%, indicating that commercializable tablets were able to be prepared.

**[Table 3]**

| Categor y | Hardness (kp) (average±stand ard deviation) | Category | Hardness (kp) (average±stand ard deviation) | Category | Hardness (kp) (average±standa rd deviation) |
|---|---|---|---|---|---|
| Example 1 | 9.5 ± 0.8 | Example 6 | 14.3 ± 0.6 | Example 11 | 13.2 ± 1.2 |
| Example 2 | 9.6 ± 1.0 | Example 7 | 10.0 ± 1.4 | Example 12 | 14.8 ± 0.9 |
| Example 3 | 10.5 ± 1.1 | Example 8 | 12.9 ± 1.2 | Example 13 | 9.9 ± 1.3 |
| Example 4 | 10.1 ± 1.0 | Example 9 | 14.4 ± 0.7 | Example 14 | 12.8 ± 1.2 |
| Example 5 | 13.1 ± 1.0 | Example 10 | 10.3 ± 1.3 | Example 15 | 14.2 ± 0.8 |

### Experimental Example 2. Comparison of Dissolution Rate for Pharmaceutical Composition Including Acid-Sensitive Ingredient and Osmotic Ingredient

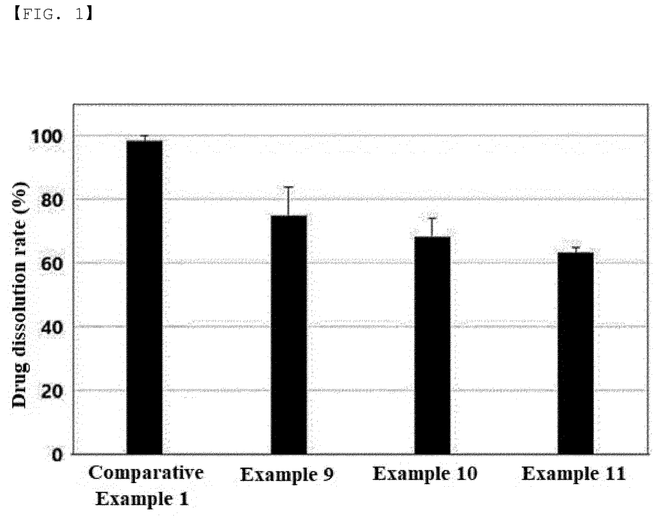

After performing a dissolution test for 2 hours in 900 mL of a pH 1.2 dissolution medium using a Korean Pharmacopoeia Dissolution Test Method (Method 2) with a dissolution tester, dissolution rates of tofacitinib citrate, the active ingredient in Example 4 to Example 6 and Comparative Example 1, were compared. The number of measured samples was 4 tables each, and the measurement results are shown in FIG. 1. As shown in FIG. 1, Example 4 to Example 6 had a decreased dissolution rate compared to Comparative Example 1, showing that the dissolution rate was controlled by the osmotic coating layer. In addition, the dissolution rate decreased as the weight of the coating layer increased with respect to the core layer among the constituents. Accordingly, it was seen that the dissolution rate was able to be controlled by adjusting the ratio of the coating layer with respect to the core layer.

### Experimental Example 3. Comparison of Table Shapes for Example 4 and Comparative Example 2 before/after Dissolution Test

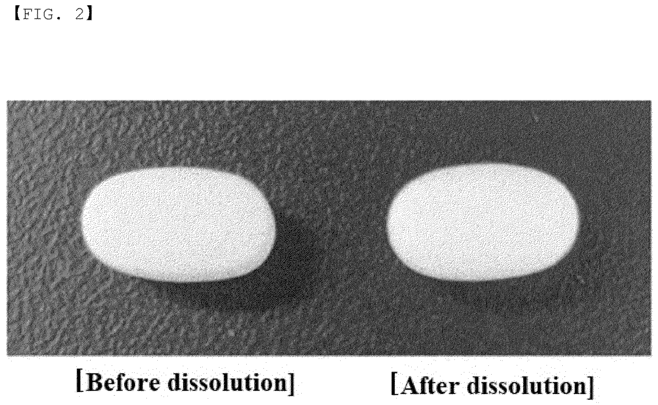

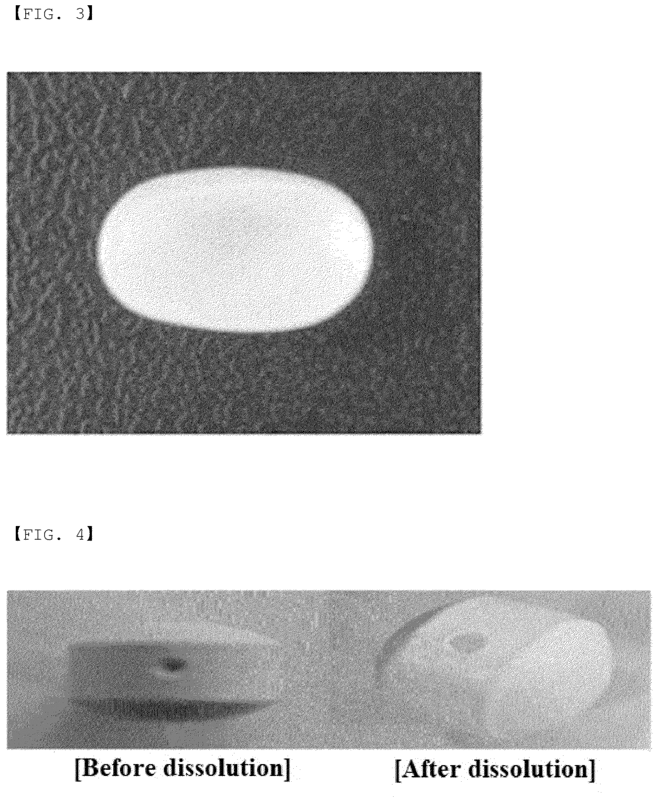

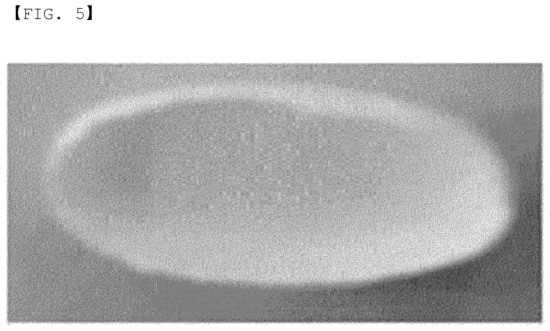

After performing a dissolution test for 12 hours on Example 4 and Comparative Example 2 in 900 mL of a pH 1.2 dissolution medium using a Korean Pharmacopoeia Dissolution Test Method (Method 2) with a dissolution tester, the shapes of Example 4 and Comparative Example 2 were compared. It was identified that the shape of the coating layer of the tablet was maintained after completing the dissolution test as shown in FIG. 2 and FIG. 4, and the core layer of the tablet was all dissolved and only the coating layer remained as shown in FIG. 3 and FIG. 5.

### Experimental Example 4. Comparison of Tablet Surfaces for Example 4 before/after Performing Dissolution Test

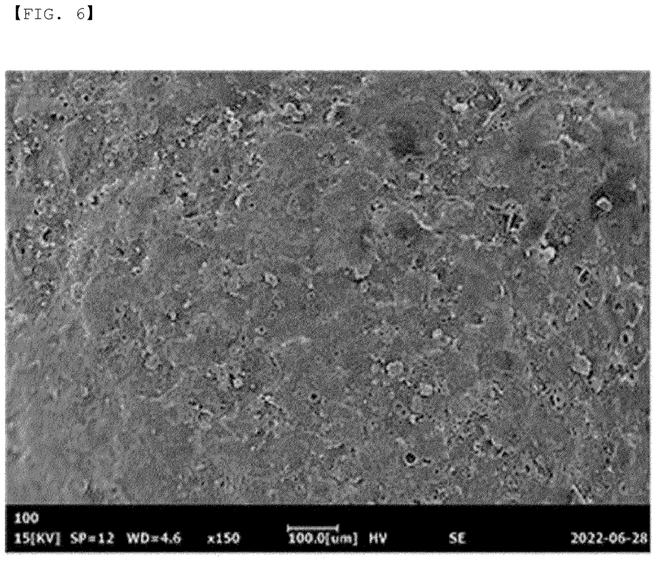

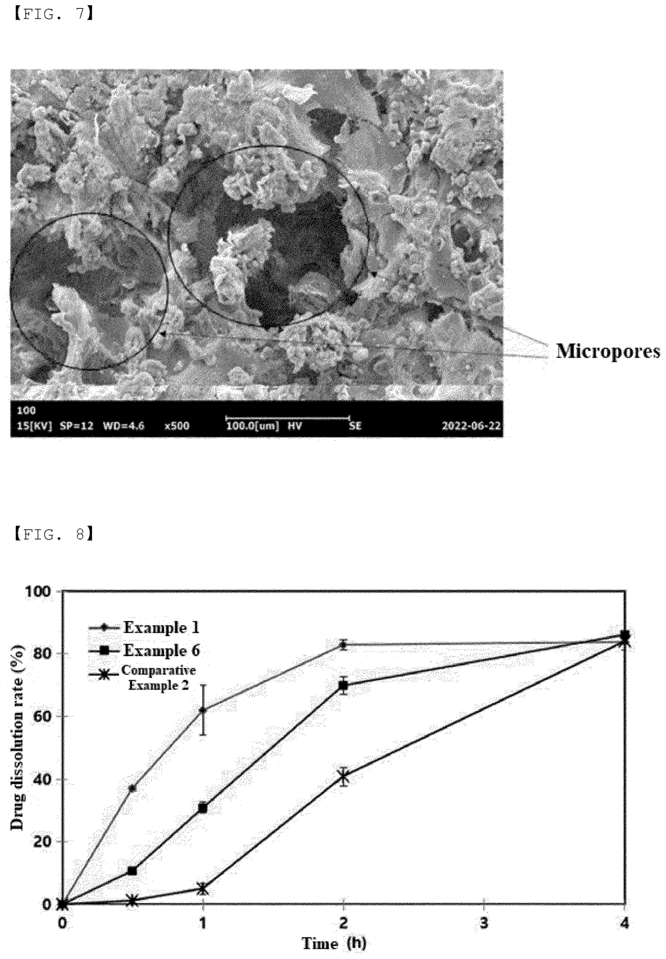

After performing a dissolution test for 12 hours on Example 4 in 900 mL of a pH 1.2 dissolution medium using a Korean Pharmacopoeia Dissolution Test Method (Method 2) with a dissolution tester, the surface conditions of the tablet before and after performing the dissolution test were compared and observed using a scanning electron microscope (SEM). As shown in FIG. 6, it was identified that the coating layer of the tablet before performing the dissolution test was a closed surface without pores. On the other hand, as shown in FIG. 7, it was identified that the tablet surface after performing the dissolution test had the acid-sensitive ingredient dissolved and had pores formed in that area.

### Experimental Example 5. Comparison of Dissolution Patterns for Example 1, Example 3 and Comparative Example 2

Dissolution patterns of tofacitinib citrate, the active ingredient in Example 1, Example 3 and Comparative Example 2, were compared for 12 hours in 900 mL of a pH 1.2 dissolution medium using a Korean Pharmacopoeia Dissolution Test Method (Method 2) with a dissolution tester. The number of measured samples was 4 tables each, and the measurement results are shown in FIG. 8.

As shown in FIG. 8, Example 3 had a similar dissolution rate in overall after the dissolution lag time compared to Comparative Example 2 that is a commercially available osmotic formulation, however, whereas the average dissolution rate within 30 minutes was 1.3% in Comparative Example 2, the rate was 37.1% in Example 1 and 10.8% in Example 3, indicating that all Examples had 2% or more of the active ingredient dissolved, and no delay in dissolution occurred after 30 minutes. On the other hand, it was seen that a delay in dissolution occurred within the first 1 hour in Comparative Example 2. Accordingly, it was identified that the pharmaceutical composition including an acid-sensitive ingredient and an osmotic ingredient of the present invention improved disadvantages of existing formulations by minimizing the initial delay in dissolution seen in Comparative Example 2.

### Experimental Example 6. Comparison of Dissolution Patterns for Tofacitinib Citrate Depending on Dissolution Medium

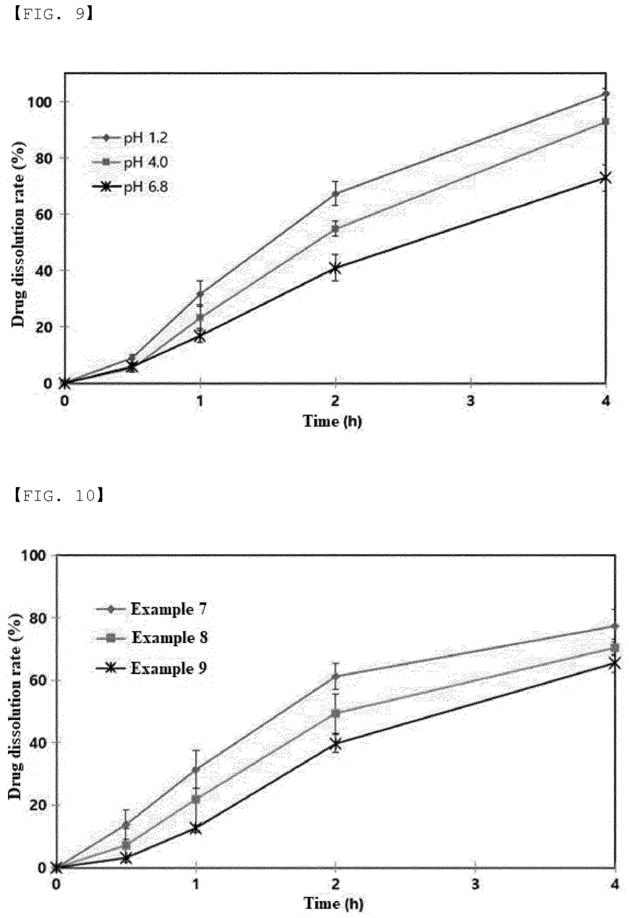

Dissolution patterns of tofacitinib citrate, the active ingredient in Example 2, were measured in 900 mL of each dissolution medium of pH 1.2, pH 4.0 and pH 6.8 using a Korean Pharmacopoeia Dissolution Test Method (Method 2) with a dissolution tester. The number of measured samples was 4 tables each, and the measurement results are shown in FIG. 9.

As shown in FIG. 9, tofacitinib citrate that is the active ingredient showed a dissolution pattern approaching a zero-order dissolution rate regardless of the dissolution medium after the dissolution lag time under an acidic condition of lower than pH 7. However, due to the acid-sensitive ingredient among the constituents, the dissolution rate tended to increase as the pH decreased, and since a delay in dissolution did not occur after 30 minutes, it was identified that an initial delay in dissolution of existing formulations was minimized.

### Experimental Example 7. Comparison of Dissolution Patterns for Nintedanib Esylate Depending on Dissolution Medium

Dissolution patterns of nintedanib esylate, the active ingredient in Example 7 to Example 9, were measured in 900 mL of a pH 1.2 dissolution medium using a Korean Pharmacopoeia Dissolution Test Method (Method 2) with a dissolution tester. The number of measured samples was 4 tables each, and the measurement results are shown in FIG. 10. As shown in FIG. 10, the dissolution rate decreased as the weight of the coating layer increased with respect to the core layer among the constituents. Accordingly, it was seen that the dissolution rate was able to be controlled by adjusting the ratio of the coating layer with respect to the core layer. In addition, nintedanib esylate that is the active ingredient showed a dissolution pattern approaching a zero-order dissolution rate regardless of the dissolution medium after the dissolution lag time, and since a delay in dissolution did not occur after 30 minutes, it was identified that an initial delay in dissolution of existing formulations was minimized.

### Experimental Example 8. Comparison of Dissolution Patterns for Apremilast Depending on Dissolution Medium

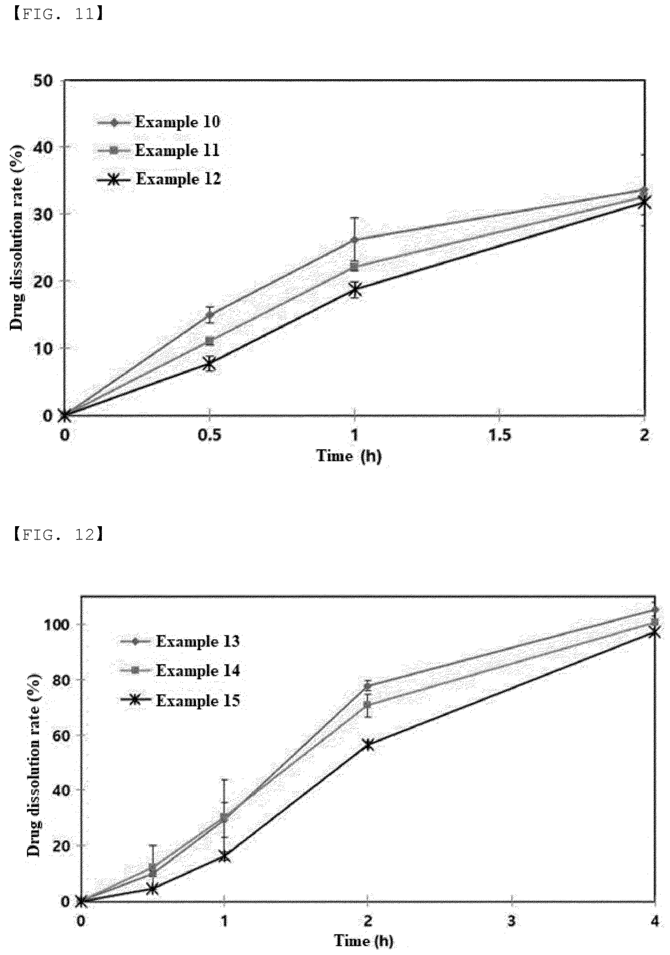

Dissolution patterns of apremilast, the active ingredient in Example 10 to Example 12, were measured in 900 mL of a pH 4.0 (including 1% sodium lauryl sulfate) dissolution medium using a Korean Pharmacopoeia Dissolution Test Method (Method 2) with a dissolution tester. The number of measured samples was 4 tables each, and the measurement results are shown in FIG. 11. As shown in FIG. 11, the dissolution rate decreased as the weight of the coating layer increased with respect to the core layer among the constituents. Accordingly, it was seen that the dissolution rate was able to be controlled by adjusting the ratio of the coating layer with respect to the core layer. In addition, apremilast that is the active ingredient showed a dissolution pattern approaching a zero-order dissolution rate regardless of the dissolution medium after the dissolution lag time, and since a delay in dissolution did not occur after 30 minutes, it was identified that an initial delay in dissolution of existing formulations was minimized.

### Experimental Example 9. Comparison of Dissolution Patterns for Pirfenidone Depending on Dissolution Medium

Dissolution patterns of pirfenidone, the active ingredient in Example 13 to Example 15, were measured in 900 mL of a pH 1.2 dissolution medium using a Korean Pharmacopoeia Dissolution Test Method (Method 2) with a dissolution tester. The number of measured samples was 4 tables each, and the measurement results are shown in FIG. 12. As shown in FIG. 12, pirfenidone that is the active ingredient showed a dissolution pattern approaching a zero-order dissolution rate regardless of the dissolution medium after the dissolution lag time, and since a delay in dissolution did not occur after 30 minutes, it was identified that an initial delay in dissolution of existing formulations was minimized.

## Claims

1. An osmotic agent composition comprising:
a core layer including an active ingredient, an osmagent and a polymer; and
a coating layer including a semi-permeable membrane coating ingredient and an acid-sensitive polymer.

2. The osmotic agent composition of claim 1, further comprising an organic acid.

3. The osmotic agent composition of claim 1, wherein the acid-sensitive polymer is an amino methacrylate copolymer or a modified copolymer thereof.

4. The osmotic agent composition of claim 3, wherein the amino methacrylate copolymer is Eudragit^{ⓡ} E.

5. The osmotic agent composition of claim 1, wherein the acid-sensitive polymer is included in an amount of 10% by weight to 90% by weight with respect to a total weight of the coating layer.

6. The osmotic agent composition of claim 2, wherein the organic acid is one or more types of pharmaceutically acceptable organic acids selected from the group consisting of citric acid, fumaric acid, tartaric acid, maleic acid, malic acid, succinic acid, oxalic acid, malonic acid, benzoic acid, mandelic acid, glutamic acid, ascorbic acid and hydrates thereof.

7. The osmotic agent composition of claim 2, comprising the organic acid and the acid-sensitive polymer in a ratio of 3:1 to 1:10.

8. The osmotic agent composition of claim 1, which has 2% or more of the active ingredient dissolved within 30 minutes in 900 mL of a pH 1.2 dissolution medium using a Korean Pharmacopoeia Dissolution Test Method (Method 2).

9. The osmotic agent composition of claim 1, which does not experience a phenomenon of delay in dissolution after 30 minutes in 900 mL of a pH 1.2 dissolution medium using a Korean Pharmacopoeia Dissolution Test Method (Method 2).

10. The osmotic agent composition of claim 1, wherein the semi-permeable membrane coating ingredient is one or more types of ingredients selected from the group consisting of cellulose acylate, cellulose diacylate, cellulose triacylate, cellulose acetate, cellulose diacetate, acetaldehyde dimethyl cellulose acetate, cellulose acetate ethyl carbamate and cellulose dimethylamino acetate.

11. The osmotic agent composition of claim 1, wherein the semi-permeable membrane coating ingredient is included in an amount of 10% by weight to 90% by weight with respect to a total weight of the coating layer.

12. The osmotic agent composition of claim 1, wherein the osmagent is one or more types of osmagent ingredients selected from the group consisting of water-soluble salts, sugars, organic acids and gelling agents.

13. The osmotic agent composition of claim 12, wherein the water-soluble salt is one or more types of ingredients selected from the group consisting of magnesium sulfate, magnesium chloride, calcium chloride, sodium chloride, lithium chloride, potassium sulfate, sodium carbonate, sodium sulfite, lithium sulfate, potassium chloride and sodium sulfate.

14. The osmotic agent composition of claim 1, wherein the osmagent is included in an amount of 1% by weight to 70% by weight with respect to a total weight of the core layer.

15. The osmotic agent composition of claim 1, wherein the polymer is one or more types of ingredients selected from the group consisting of sodium carboxymethyl cellulose, carboxymethyl cellulose, hydroxypropyl cellulose, hypromellose, methyl cellulose, hydroxyethyl cellulose, polyvinyl pyrrolidone, a vinyl pyrrolidone and vinyl acetate copolymer, polyethylene oxide, ethyl cellulose and wax.

16. The osmotic agent composition of claim 1, further comprising one or more types of moisture absorbing components selected from the group consisting of colloidal silicon dioxide, polyvinyl pyrrolidone, sodium lauryl sulfate, kaolin, bentonite, niacinamide, alumina and titanium dioxide.

17. The osmotic agent composition of claim 1, further comprising one or more types of dissolution enhancing agents selected from the group consisting of polyvinyl pyrrolidone, polyethylene glycol, beta-cyclodextrin, polysorbate, sodium lauryl sulfate, alkyl ester, sorbitan monolaurate, sorbitan monostearate and sorbitan monopalmitate.

18. The osmotic agent composition of claim 1, further comprising one or more types of plasticizers selected from the group consisting of polyethylene glycol, triacetin, triethyl citrate, dibutyl sebacate, ethylene glycol monoacetate, ethylene glycol diacetate, triethyl phosphate and diethyl tartrate.

19. The osmotic agent composition of claim 1, further comprising a diluting agent, a binding agent, a disintegrating agent, a foaming agent, an emulsifying agent, a pH regulating agent, a lubricant or a glidant component.

20. The osmotic agent composition of claim 1, which has a coating layer thickness of 1 micrometer to 1,000 micrometers.

21. The osmotic agent composition of claim 1, wherein the active ingredient is an ingredient selected from the group consisting of tofacitinib, apremilast, nintedanib and pirfenidone, or a pharmaceutically acceptable salt thereof.

22. The osmotic agent composition of claim 1, which has no visually identifiable micropores before administration.
